(19) **Europäisches Patentamt / European Patent Office / Office européen des brevets**

(11) **EP 1 351 713 B1**

(12) # EUROPEAN PATENT SPECIFICATION

(45) Date of publication and mention of the grant of the patent:
**15.10.2008 Bulletin 2008/42**

(51) Int Cl.:
*A61K 51/10* (2006.01)    *A61K 39/395* (2006.01)
*C07K 16/28* (2006.01)    *C07K 16/30* (2006.01)

(21) Application number: **01996085.5**

(22) Date of filing: **24.10.2001**

(86) International application number:
**PCT/US2001/046104**

(87) International publication number:
**WO 2002/051448 (04.07.2002 Gazette 2002/27)**

(54) **ANTI-TENASCIN MONOCLONAL ANTIBODY THERAPY FOR LYMPHOMA**

LYMPHOMBEHANDLUNG MIT DEM MONOKLONALEN ANTI-TENASCIN-ANTIKÖRPER

THERAPIE A BASE D'ANTICORPS MONOCLONAL ANTI-TENASCINE POUR LE TRAITEMENT DES LYMPHOMES

(84) Designated Contracting States:
**AT BE CH CY DE DK ES FI FR GB GR IE IT LI LU MC NL PT SE TR**

(30) Priority: **21.12.2000 US 257108 P**

(43) Date of publication of application:
**15.10.2003 Bulletin 2003/42**

(73) Proprietor: **Duke University Durham, North Carolina 27708-0083 (US)**

(72) Inventors:
- **RIZZIERI, David**
  **Chapel Hill, NC 27514 (US)**
- **BIGNER, Darell, D.**
  **Mebane, NC 27302 (US)**
- **ZALUTSKY, Michael**
  **Chapel Hill, NC 27514 (US)**

(74) Representative: **Horner, Martin Grenville et al Marks & Clerk 19 Royal Exchange Square Glasgow G1 3AE (GB)**

(56) References cited:
**WO-A-92/04464       US-A- 5 624 659**

- **KAMINSKI MARK S ET AL:** "Radioimmunotherapy with iodine 131I tositumomab for relapsed or refractory B-cell non-Hodgkin lymphoma: Updated results and long-term follow-up of the University of Michigan experience" BLOOD, vol. 96, no. 4, 15 August 2000 (2000-08-15), pages 1259-1266, XP002334896 ISSN: 0006-4971
- **BIGNER D D ET AL:** "Iodine-131-labeled antitenascin monoclonal antibody 81C6 treatment of patients with recurrent malignant gliomas: phase I trial results." JOURNAL OF CLINICAL ONCOLOGY : OFFICIAL JOURNAL OF THE AMERICAN SOCIETY OF CLINICAL ONCOLOGY. JUN 1998, vol. 16, no. 6, June 1998 (1998-06), pages 2202-2212, XP002334897 ISSN: 0732-183X
- **CHILOSI M. ET AL.:** 'Constitutive expression of tenascin in T-dependent zones of human lymphoid tissues' AM. J. PATHOL. vol. 143, no. 5, November 1993, XP002908626
- **DATABASE BIOSIS [Online] XP002908627** Database accession no. 2002:152427 & RIZZIERI D.A. ET AL.: 'Radiolabeled anti-tenascin antibody for refractory non-Hodgkins lymphoma (NHL)' BLOOD vol. 98, no. 11, PART 2, 16 November 2001, page 247B
- **RIZZIERI A.D. ET AL.:** 'Markers of angiogenesis, factor VIII and tenascin correlate with disease activity in patients with non Hodgkin's lymphoma' BLOOD vol. 94, no. 10, PART 2, SUPPL. 1, 15 November 1999, page 253B #439, XP002908483

EP 1 351 713 B1

**(Cont. next page)**

- RIZZIERI D.A. ET AL: 'PHASE 1 TRIAL STUDY OF 131 I-LABELED CHIMERIC 81C6 MONOCLONAL ANTIBODY FOR THE TREATMENT OF PATIENTS WITH NON-HODGKIN LYMPHOMA' BLOOD, W.B.SAUNDERS COMPANY vol. 104, no. 3, 01 August 2004, ORLANDO, FL, US, pages 642 - 648, XP003009631

- COKGOR I. ET AL: 'Phase I trial results of iodine-131-labeled antitenascin monoclonal antibody 81C6 treatment of patients with newly diagnosed malignant gliomas.' JOURNAL OF CLINICAL ONCOLOGY : OFFICIAL JOURNAL OF THE AMERICAN SOCIETY OF CLINICAL ONCOLOGY vol. 18, no. 22, 15 November 2000, UNITED STATES, pages 3862 - 3872

**Description**

**Related Applications**

This application claims the benefit of provisional application serial number 60/257,108 filed December 21, 2000.

**Field of the Invention**

The present invention concerns medicaments for the treatment of lymphomas such as Non-Hodgkin's lymphoma.

**Background of the Invention**

Non-Hodgkin's lymphomas (NHL) are a varied group of hematologic malignancies of lymphoid origin. Patients with low grade NHL have a long survival, but are rarely cured of the disease. Patients with intermediate or high-grade lymphomas are likely to respond to conventional chemotherapy with 55 to 70 percent attaining a complete remission. Unfortunately, most relapse at a median time of 2 to 8 years depending upon the subtype of NHL with which they are afflicted. Five-year survival is only about 35 to 45 percent. Accordingly, new approaches to treating lymphoma must be developed to improve the outcome for most patients.

U.S. Patent No. 5,624,659 to Bigncr and Zalutsky describes methods of treating solid and cystic tumors with monoclonal antibody 81CS. However, the treatment of lymphoma is neither suggested nor disclosed in this patent.

Rizzieri et al. (Blood, Vol. 94(10), part 2, supp. 1. 1999-11-15) suggests the use of anti-tenascin antibodies delivered systemically to treat non Hodgkin's lymphoma, however it does not suggest using antibodies coupled to a radioisotope.

**Summary of the Invention**

A first aspect of the present invention is a use of an antibody that binds to tenascin, for the manufacture of a medicament for the treatment of non Hodgkins lymphoma by parenteral administration:

the antibody bein selected from monoclonal antibody 81C6 and antibodies that bind to the epitope bound by monoclonal antibody 81C6; and
the antibody being coupled to a radioisotope. The antibody-radioisotope conjugate may be administered at any suitable dose, such as a dose of from 50 to 100 Gy (5,000 rads to 100,000 rads).

In a preferred embodiment, the antibody that binds to tenascin is used to treat lymphomas that are unresponsive to conventional chemotherapy regimes.

The foregoing and other objects and aspects of the present invention are explained in detail in the drawings herein and the specification set forth below.

**Detailed Description of the Preferred Embodiment**

Subjects that may be treated by the medicaments of the present invention include both human subjects for medical purposes and animal subjects for veterinary and drug screening and development purposes. Suitable animal subjects include both avians and mammals, with mammals being preferred. The term "avian" as used herein includes, but is not limited to, chickens, ducks, geese, quail, turkeys and pheasants. The term "mammal" as used herein includes, but is not limited to, primates, bovines, ovines, caprines, porcines, equines, felines, canines, lagomorphs, rodents (*e.g.*, rats and mice), *etc*. Human subjects are the most preferred. Human subjects include fetal neonatal, infant, juvenile and adult subjects.

The terms "monoclonal antibody 81C6", "antibody 81C6", or similar terms encompass both the murine monoclonal antibody 81 C6 and the humanized chimeric antibody 81C6, both of which are described in U.S. Patent No. 6,624,659.

The term "treat" as used herein refers to any type of treatment or prevention that imparts a benefit to a subject afflicted with a disease or at risk of developing the disease, including improvement in the condition of the subject (*e.g.*, in one or more symptoms), delay in the progression of the disease, delay the onset of symptoms or slow the progression of symptoms, etc. As such, the term "treatment" also includes prophylactic treatment of the subject to prevent the onset of symptoms. As used herein, "treatment" and "prevention" are not necessarily meant to imply cure or complete abolition of symptoms." to any type of treatment that imparts a benefit to a patient afflicted with a disease, including improvement in the condition of the patient (*e.g.*, in one or more symptoms), delay in the progression of the disease, etc.

The term "treatment effective amount" as used herein means an amount of the inventive antibody sufficient to produce a desirable effect upon a patient inflicted with lymphoma, including improvement in the condition of the patient (*e.g.*, in one or more symptoms), delay in the progression of the disease, etc.

The term "pharmaceutically acceptable" as used herein means that the compound or composition is suitable for administration to a subject to achieve the treatments described herein, without unduly deleterious side effects in light of the severity of the disease and necessity of the treatment.

### A. lymphoma Subjects.

The subject may be any subject carrying or afflicted with, or suspected of carrying or being afflicted with, lymphoma. The Lymphomas that may be treated by the medicaments of the present invention are Non-Hodgkin's lymphoma Examples of Non-Hodgkin's lymphomas include those identified in 1982 by the Non-Hodgkin's Pathological Classification Project as an "international Working Formulation" (IWF) and set forth in **Table 1** below. Additionally, these subjects may include those subjects who are partially or completely unresponsive to treatment using other chemotherapy regimens (*e.g.*, cyclophoaphamide, doxorabicine vincristine, prednisone, etc.), including subjects that are partially or completely unresponsive to rituximab therapy.

**Table 1: Classification schema and survival of patients with non-Hodgkin's lymphoma.[1]**

| Prognostic Group | Type | Percent | Median Survival (yrs) |
|---|---|---|---|
| **Low grade-** IWF A, | small lymphocytic | 3.6 | 5.8 |
| IWF B | follicular, small cleaved | 22.5 | 7.2 |
| IWF C | follicular, mixed small and large cell | 7.7 | 5.1 |
| **Intermediate grade** IWF D | follicular, large cell | 3.8 | 3.0 |
| IWF E | diffuse, small cleaved | 6.9 | 3.4 |
| IWF F | diffuse, mixed small and large cell | 6.7 | 2.7 |
| IWF G | diffuse, large cell | 19.7 | 1.5 |
| **High grade-** IWFH | large cell, immunoblastic | 7.9 | 1.3 |
| | lymphoblastic | 4.2 | 2.0 |
| | small, noncleaved | 5.0 | .7 |
| **Miscellaneous** | | 12 | |

[1]Non-Hodgkin's Lymphoma Pathologic .Classification Project. National Cancer Institute sponsored study of classification of Non-hodgkin's lymphomas. Cancer 49: 2112-2135 (1982).

### B. Antibodies.

The term "antibodies" as used herein refers to all types of immunoglobulins, including IgG, IgM, IgA, IgD, and IgE. The term "immunoglobulin" includes the subtypes of these immunoglobulins, such as $IgG_1$, $IgG_2$, $IgG_3$, $IgG_4$, etc. Of these immunoglobulins, ZgM and IgG are preferred, and IgG is particularly preferred. The antibodies may be of any species of origin, including (for example) mouse, rat, rabbit, horse, or human, or may be chimeric antibodies. See, *e.g.*, M. Walker et al., Molec. Immunol. 26, 403-11 (1989). Such Monoclonal antibodies are produced in accordance with known techniques. The term "antibody" as used herein includes antibody fragments which retain the capability of binding to a target antigen, for example, Fab, $F(ab')_2$, and Fv fragments, and the corresponding fragments obtained from antibodies other than IgG. Such fragments are also produced by known techniques.

The monoclonal antibodies may be recombinant monoclonal antibodies produced according to the methods disclosed in Reading U.S. Pat. No. 4,474,893, or Cabilly et al., U.S. Pat. No. 4,816,567. The antibodies may also be chemically constructed by specific antibodies made according to the method disclosed in Segel et al., U.S. Pat. No. 4,676,980.

Monoclonal antibodies may be chimeric antibodies produced in accordance with known techniques. For example, chimeric monoclonal antibodies may be complementarily determining region-grafted antibodies (or "CDR-grafted antibodies") produced in accordance with known techniques.

Monoclonal Fab fragments may be produced in *Escherichia coli* by recombinant techniques known to those skilled in the art. See, e.g., W. Huse, Science 246,1275-81 (1989).

As noted above, antibodies employed in carrying out the present invention are those which bind to tenascin. Particularly preferred are monoclonal antibody 81C6 and antibodies that bind to the epitope bound by monoclonal antibody 81C6 (i.e., antibodies that cross-react with, or block the binding of, monoclonal antibody 81C6). The monoclonal antibody 81C6 is a murine IgG2b monoclonal antibody raised from a hybridoma fusion following immunization of BALB/c mice with the glial fibrillary acidic protein (GFAP)-expressing permanent humanglioma line U-251 MG, as known and described in M. Bourdon et al., Cancer Res 43, 2796 (1983).

Particularly preferred for carrying out the present invention is a mouse-human chimeric monoclonal antibody 81C6, as described in U.S. Patent No. 5,624,659 to Bigner and Zalutsky.

Antibodies for use in the present invention specifically bind to tenascin with a relatively high binding affinity, for example, with a dissociation constant of about $10^{-4}$ to $10^{-13}$. In embodiments of the invention, the dissociation constant of the antibody-tenascin complex is at least $10^{-4}$, preferably at least $10^{-6}$, and more preferably at least $10^{-9}$.

Monoclonal antibodies used for the medicaments described herein may be monoclonal antibodies coupled to a therapeutic agent radioisotope. See generally Monoclonal Antibodies and Cancer Therapy (R. Reisfeld and S. Sell Eds. 1985)(Alan R. Liss Inc. N.Y.). Therapeutic agents such as radioisotopes may be coupled to the antibody by direct means or indirect means (e.g., via a chelator) by any suitable technique, such as the Iodogen method or with N-succinimidyl-3-(tri-n-butylstanyl)benzoate (the "ATE method"), as will be apparent to those skilled in the art *See, e.g.,* M. Zalutsky and A. Narula, Appl. Radiat. Isot. 38, 1051 (1987).

Examples of radioisotopes which may be coupled to a therapeutic monoclonal antibody include, $^{131}$I, $^{90}$Y, $^{211}$At, $^{212}$Bi, $^{67}$Cu, $^{186}$Re, $^{188}$Re, and $^{212}$Pb.

It will be appreciated that monoclonal antibodies per se which are used as therapeutic monoclonal antibodies incorporate those portions of the constant region of an antibody necessary to evoke a therapeutically useful immunological response in the subject being treated.

## C. Formulations and administration.

For administration of the medicament, the antibody will generally be mixed, prior to administration, with a nontoxic, pharmaceutically acceptable carrier substance (e.g. normal saline or phosphate-buffered saline), and will be administered by parenteral administration (*e.g.*, injection) such as by intravenous or intra-arterial injection.

The antibody active compounds described above may be formulated for administration in a pharmaceutical carrier in accordance with known techniques. *See, e.g.*, Remington, The Science And Practice of Pharmacy (9th Ed. 1995). In the manufacture of a pharmaceutical formulation according to the invention, the active compound (including the physiologically acceptable salts thereof) is typically admixed with, *inter alia*, an acceptable carrier. The carrier must, of course, be acceptable in the sense of being compatible with any other ingredients in the formulation and must not be deleterious to the patient The carrier may be a liquid and is preferably formulated with the compound as a unit-dose formulation which may contain from 0.01 or 0.5% to 95% or 99% by weight of the active compound.

As discussed below, the medicaments of the present invention may optionally be administered in conjunction with other, different, active compounds useful in the treatment of the disorders or conditions described herein (e.g., chemotherapeutics). The other compounds may be administered concurrently. As used herein, the word "concurrently" means sufficiently close in time to produce a combined effect (that is, concurrently may be simultaneously, or it may be two or more administration occurring before or after each other).

Formulations of the present invention suitable for parenteral administration comprise sterile aqueous and non-aqueous injection solutions of the active compound, which preparations are preferably isotonic with the blood of the intended recipient. These preparations may contain anti-oxidants, buffers, bacteriostats and solutes which render the formulation isotonic with the blood of the intended recipient

The medicament may be administered by any medically appropriate procedure, e.g., normal intravenous or intra-arterial administration, injection into the cerebrospinal fluid). In certain cases, intradermal, intracavity, intrathecal or direct administration to the tumor or to an artery supplying the tumor is advantageous.

Therapeutic monoclonal antibodies may be provided in lyophylized form in a sterile aseptic container or may be provided in a pharmaceutical formulation in combination with a pharmaceutically acceptable carrier, such as sterile pyrogen-free water or sterile pyrogen-free physiological saline solution.

Dosage of the medicament will depend, among other things, the condition of the subject, the particular category or type of cancer being treated, the route of administration, the nature of the therapeutic agent employed, and the sensitivity of the tumor to the particular therapeutic agent For example, the dosage will typically be about 1 to 10 micrograms per kilogram subject body weight. The specific dosage of the medicament is not critical, as long as it is effective to result in some beneficial effects in some individuals within an affected population. In general, the dosage may be as low as about 0.05, 0.1, 0.5, 1, 5, 10, 20 or 50 micrograms per kilogram subject body weight, or lower, and as high as about 5, 10, 20, 50, 75 or 100 micrograms per kilogram subject body weight, or even higher.

In another example, where the therapeutic agent is $^{131}$I, the dosage to the patient will typically be from 10 mCi to 100, 300 or even 500 mCi. Stated otherwise, where the therapeutic agent is $^{131}$I, the dosage to the patient will typically be from 50 to 1000 Gy (5,000 Rads to 100,000 Rads) (preferably at least 130 Gy (13,000 Rads), or even at least 500 Gy (50,000 Rads)). Doses for other radionuclides are typically selected so that the tumoricidal dose will be equivalent to the foregoing range for $^{131}$I. The medicament can be administered to the subject in a series of more than one administration, and regular periodic administration will sometimes be required.

The present invention is explained in greater detail in the Examples set forth below. In the Examples "sski" means saturated solution of potassium iodide, "P.O." means by mouth, "b.i.d." means twice daily, "t.i.d." means three times daily, and "q.d." means daily.

## EXAMPLES 1

### Formulation and Route of Administration

The drug (antibody) is supplied in a 2 milliliter sterile, pyrogen-free solution that contains 10 mg chimeric 81C6 antibody ("ch81c6") (20-60 mCi $^{131}$I), 0.7 to 0.9% sodium chloride, 0 to 0.6% sodium phosphate, 0.5% albutein, and water. It is prepared within 24 hours of its use.

The drug described above is administered intravenously. The patient receives premedication with diphenhydramine (50 mg IV) and acetaminophen (650 mg orally) 30 to 60 minutes before the antibody is given. The unlabeled antibody dose is given intravenously over 60 minutes (longer if side effects are noted and the infusion is slowed down). The labeled antibody is delivered over a time of 30 minutes. The unlabeled and radiolabeled antibody is delivered via an intravenous catheter (PIC line or central venous catheter). An in-line filter is used with infusion of 0.22 micron filter size. Vital signs are monitored every 15 minutes during the infusion and for 1 hour afterwards.

### EXAMPLE 2

### Treatment Procedure

*Peripheral Blood Progenitor Cell (PBPC) Harvest (Stem cell back-up).*

Patients receive 10 mcg/kg, sc daily throughout the PBPC collection. G-CSF doses are rounded off to use nearest whole vials (300 mcg or 480 mcg). Leukopheresis begins after 3 days of GCSF treatment. Patients are continued on four - six hour, daily apheresis collections for up to 4 days or until a total of $\geq 1 \times 10^8$ mononuclear cells/kg is achieved. In the uncommon event that adequate PBPC are not collected, the treating physician may choose to proceed with bone marrow harvest to collect the same goal of cells for the patient's backup (the total cell count is a combination of peripheral blood and marrow collected).

*Antibody therapy.* Thyroid suppression beginning at 48 hours prior to treatment and continuing for 28 days after the therapeutic dose (may be given with juice or soda to mask the taste) with: (1) SSKI 2 drops P.O. b.i.d., and Cytomel 75 mcg P.O. q.d., or (2) potassium iodide tablets 130 mg P.O. q.d., or (3) Lugol's solution 20 drops P.O. t.i.d. Preferably patients do not receive any radiolabeled antibody doses before they have completed at least 24 hours of therapy with 1 of the above medication regimens.

Patients are first given a varying dose of unlabeled ch81 C6 (0 mg, 50 mg, 100 mg, 200 mg, or 400 mg) followed by a tracer amount of 10 mCi (millicuries) of $^{131}$I on a constant dose of 10 mg of ch81 C6 Mab. This is termed the "dosimetric dose" of the patient's therapy. Because of slight variations in yield and transfer of radiolabeled protein to injection syringes, the administered dose is 10 mg $\pm$ 2 mg. The administered radiation dose for the same reasons may vary 10% from the targeted dose. Patients have blood drawn for pharmacokinetic measurements after the dosimetric dose at time 6 hours, 24 hours, and every 24 hours through 7 days after the dose is given. Patients have Gamma camera imaging with whole body planar and SPECT analyses at 24 hours, and every 24 hours for 7 days. From this data, the activity of the $^{131}$I labeled ch81C6 is determined and the "therapeutic dose" in milliCuries calculated, which provides the goal dose of radiation. The total body goal dose of radiation is 75 Gy, unless the platelet count is less than 100,000/ml in which case the goal total body radiation dose is 65 Gy.

The patient returns (approximately 1-2 weeks after the initial dose) for the "therapeutic dose" infusion. The patient generally receives the same dose of cold or unlabeled antibody they received for the dosimetric dose, and the therapeutic dose of radiolabel is on a constant dose of 10 mg of Ch81 C6 monoclonal antibody. The lesser of the "calculated whole body mass" or actual mass in kilograms is used for the radiation dose.

## EXAMPLE 3

### Clinical Trial Protocol

The toxicities of the intravenously delivered antibody and the optimal dose of cold antibody to be given prior to the radiolabeled dose to maximize localization to the tumor are evaluated. Patients are closely monitored for toxicity and have pharmacokinetic and dosimetric analyses performed. Further, a back-up stem cell or marrow collection is required for all patients.

This trial does allow those who have had prior radiation or organ damage to be entered onto this trial as each patient's dose is individualized. The accepted organ tolerances are used to ensure the calculated safe radiation doses do not exceed these tolerances for each patient.

### Eligibility:

1. Patients with relapsed or refractory, biopsy proven, NHL. Patients should have failed or progressed after at least 1 regimen of chemotherapy. Patients must not be considered eligible for other standard approaches with curative intent (i.e. autologous bone marrow transplantation for first relapse patients with chemotherapy sensitive disease).

2. Pregnant or lactating women are not eligible. Women with child bearing potential will have a serum B-HCG tested within 72 hours of initiation of chemotherapy. Further, patients must agree to use an effective form of birth control for the duration of time they are receiving therapy on this protocol. Medically accepted forms of contraceptives include (1) surgical sterilization, (2) approved hormonal contraceptives (such as birth control pills, Depo-Provera, or Dupolupron), (3) barrier methods used with spermicides or (4) intrauterine devices.

3. Age $\geq$ 18 years of age.

4. No significant medical and/or psychiatric illness which, in the opinion of the investigators, may compromise any aspect of the planned treatment.

5. Minimum WBC 4.0 with absolute neutrophil count >1000, platelet count >100k, hematocrit 30% pre-therapy.

6. AST/ALT, alkaline phosphatase and total bilirubin < 2.0x normal control. Patients with >25% of the liver involved with disease are not eligible.

7. Estimated Creatinine clearance > 60 mg/ml

$$\text{estimated creatinine clearance} = \frac{(140 - \text{age})\,(\text{weight in Kg})}{72 \times \text{serum Cr}}\ \{\text{female multiply by .85}\}$$

*Patients may not have active obstructive hydronephrosis.

8. CALGB performance status $\leq$ 3.

9. Estimated life expectancy of at least 3 months.

10. Ability to collect adequate stem cells or marrow for 'back -up' for all patients. A total of >= 1 x $10^8$ mononuclear cells /kg is required.

11. Patients must be HIV negative (may be tested up to 3 weeks in advance).

12. Patients must not have active viral (A, B, or C) or auto-immune hepatitis by screening or history.

13. Patients may not have undergone prior high dose chemotherapy that required bone marrow or stem cell support (allogeneic or autologous).

14. Patients must have less than 25% of the marrow involved with lymphoma to be eligible (if only a unilateral marrow is done, then <10% of the marrow may be involved).

15. Ability to safely radiate the patients under the limits of dose tolerance if prior radiation or damage to visceral organs has previously occurred. The accepted doses that the patients should not have exceeded prior to consideration for this trial are as below:

Liver: 30 Gy
Stomach/colon: 20 Gy
Marrow: 65 cGy
Heart: 20 Gy

Lungs: 40 Gy
Brain: 62 Gy
Renal: 25 Gy
Thyroid 55 Gy

Patients may not have previously received radiation to a maximum dose to any organ and no more than 25% of the skeleton may have been previously radiated.

If patients have received prior radiotherapy to doses near these levels, clearance from radiation therapy physicians should be performed before entering the patient on this trial.

16. Patients with previous allergic reactions to iodine are excluded (this does NOT include prior reactions to iodine containing intravenous contrast materials).

17. Patients may not receive other concurrent anti-cancer therapies or unapproved drugs.

Clarifications:

Steroids are permitted, though should be at the lowest possible dose and at a stable dose for at least 5 days before therapy is administered.

**Treatment Evaluate:**

It is recommended that scans or x-rays used to document measurable or evaluable disease response should be done within 7 days of therapy, unless otherwise noted (to prevent the unnecessary duplication of tests, radiographic studies may be done up to 4 weeks before and blood work up to 2 weeks before if the treating team documents there has not been a significant change in the patient's status since that time and there has been no chemotherapy given in the interval). Parameters to be monitored and evaluated include:

1. Height, weight, performance status, serum B-HCG if of child bearing potential.

2. ABC/diff, Serum BUN / K / NA / CL / CO3 / Cr / Glu / AST / ALT / TB / Alk Phos/LDH/Ca/Mg/PO/urate/Alb/TP, urine analysis for proteinuria pre therapy, daily for 3 days, then at least weekly for 4 weeks or until lab values return to pretherapy levels, whichever occurs last (unless progressive disease prevents a return to pretreatment values as documented by the treating physician).

3. Bone Marrow aspirate and biopsy- Bilateral marrow is requested at entry and may be considered as part of the standard of care, however, a unilateral marrow is acceptable. A marrow at recovery from therapy (about 3 weeks after the last dose) is also required.

4. Lymph node biopsy- patients with easily accessible lymph nodes may be requested to have a biopsy of one of these nodes after therapy is completed.

5. CT of chest/abdomen/and pelvis (and other sites of known disease) pre-therapy and at recovery (if patients have had obvious progression by physical exam, these need not be repeated after therapy).

6. Gallium scan done pretherapy and at recovery post therapy if the other restaging tests indicate remission obtained. If the test was negative pre-therapy, it is not repeated at completion of therapy.

7. Thyroid panel to include TSH, $T_3$ and $T_4$ levels and at 4 weeks following therapy.

8. Two red tops .(preservative-free) and 2 purple tops (containing the preservative ethylenediaminetetraacetic acid (EDTA)) to the serum/plasma bank pretherapy, and at recovery from therapy (about 3 weeks after the dose). Tumor block or 15 unstained slides must be sent to study chair from **all** biopsies done in evaluation of the lymphoma diagnosis since initial diagnosis, pre therapy (of involved site and uninvolved site if available), and at completion of therapy (about 3 weeks after the dose) (if available), and at progression of disease (if applicable). Two milliliters of peripheral blood at 6 hours after the dosimetric dose and then at 24 hours after the dose and every 24 hours until day +7 after $^{131}$I ch81C6 dosimetric injection for scintillation counting and pharmacokinetics.

9. Gamma camera SPECT and whole body imaging will be performed 24 hours post infusion and daily for 7 days.

**Drug Information:**

**Cold and Radiolabeled Antibody to Tenascin:**

1. Formulation

The drug is supplied in a 2-ml sterile, pyrogen-free solution that contains 10 mg ch81C6 (20-60 mCi $^{131}$I), 0.7-0.9% sodium chloride, 0-0.6% sodium phosphate, 0.5% albutein, and water. It is prepared within 24 hours of its use.

2. Route of administration

Patients receive premedications with diphenhydramine (50 mg IV) and acetaminophen (650 mg orally) 30 - 60 minutes before the antibody is given. The labeled dose is administered over 30 minutes. The radiolabeled antibody is delivered via an intravenous catheter (PIC line or central venous catheter). An in-line filter may be used with infusion of 0.22 micron filter size. Vital signs should be monitored every 15 minutes during the infusion and for 1 hour afterwards. The labeled dose is escalated as noted below.

3. Anticipated toxicity

There has been little toxicity with the administration of the prior murine Mab 81C6. However, symptoms of reactions to foreign protein could include the following: chills, fever, urticaria, anaphylaxis, aseptic meningitis, hemolysis, leukopenia, thrombocytopenia and serum sickness.
In addition, iodine allergy manifested by rash, nausea, or anaphylaxis may occur. Patients are carefully monitored for all these potential complications and appropriate measures taken.
Radiation damage may include necrosis, liver failure, renal failure, lung failure, prolonged cytopenias and the need for transfusion support, decreased white cell counts leading to increased risks for infections, secondary cancers, nausea, and cataracts.

**Treatment Plan:**

**1. Peripheral Blood Progenitor Cell Harvest (Stem cell back-up):**

Patients receive 10mcg/kg, sc daily throughout the PBPC collection. G-CSF doses should be rounded off to use nearest whole vials (300mcg or 480mcg). Leukopheresis will begin after 3 days of GCSF. Patients are continued on four - six hour, daily apheresis collections for up to 4 days or until a total of $\geq 1 \times 10^8$ mononuclear cells/kg is achieved. In the uncommon event that adequate PBPC cannot be collected, the treating physician may choose to proceed with bone marrow harvest to collect the same goal dose of cells for the patient's backup (the total cell count will be a combination of peripheral blood and marrow collected).
This trial does NOT preclude the common practice of collecting stem cells upon recovery from chemotherapy that is also used for anti-lymphoma therapy by the treating physician, it just does not *require* that chemotherapy be used to collect the stem cells.

**2. Antibody therapy:**

The primary goal is to estimate the maximum tolerated dose of radiolabeled antibody that can be administered. Information is also be collected in terms of dosimetry and pharmacokinetics to determine how the total body exposure relates to the total dose delivered for future trials.
1) Thyroid suppression beginning at **48 hrs. prior** to treatment and continuing for 28 days after the therapeutic dose (may give with juice or soda to mask the taste) with:

1. SSKI 2 drops P.O. b.i.d., and Cytomel 75 mcg P.O. q.d., or
2. Potassium iodide tablets 130 mg P.O. q.d., or
3. Lugol's solution 20 drops P.O. t.i.d.
Patients should not receive any radiolabeled antibody doses before they have completed at least 24 hours of therapy with 1 of the above medication regimens.

2) Patients are enrolled and treated in a standard phase I manner for each cohort of radiolabel dosing. If zero patients experience dose limiting toxicity at a certain dose, the dose is escalated to the next level. If 1 patient experiences dose limiting toxicity of the first 3, then 3 more will be entered at the same level. If $\leq 2$ of these 6 patients experience a dose limiting toxicity, then the dose is escalated to the next cohort. However, if 2 or 3 patients of the first 3 patients experience dose limiting toxicity, then the maximum tolerated dose has been exceeded and this trial is closed. The maximum tolerated dose is estimated as the next lower dose of radiolabel in this trial (the highest dose given that did not exceed the maximum tolerated dose).

**Table 2: Radiolabeled dosing.**

| Radiolabeled antibody dose | Number treated | Number experiencing dose limiting toxicity | Action |
|---|---|---|---|
| 40 mCi | 3 | 0<br>1<br><br>2 | Escalate to next cohort<br>Continue accrual<br><br>Maximum dose exceeded. Close trial and get approval to deescalate dose |
| 40 mCi | 6 | 1 or 2<br><br>3 or more | Advance to next cohort<br><br>Maximum dose exceeded. Close trial and get approval to deescalate dose |
| 60 mCi | 3 | 0<br>1<br><br>2 | Escalate to next cohort<br>Continue accrual<br><br>Maximum dose exceeded, close trial |
| 60 mCi | 6 | 1 or 2<br><br>3 or more | Advance to next cohort<br><br>Maximum dose exceeded, close trial |
| 80 mCi | 3 | 0<br>1<br><br>2 | Escalate to next cohort<br>Continue accrual<br><br>Maximum dose exceeded, close trial |
| 80 mCi | 6 | 1 or 2<br><br>3 or more | Advance to next cohort<br><br>Maximum dose exceeded, close trial |
| 100 mCi | 3 | 0<br>1<br><br>2 | Escalate to next cohort<br>Continue accrual<br><br>Maximum dose exceeded, close trial |
| 100 mCi | 6 | 1 or 2<br><br>3 or more | Maximum rec. dose<br><br>Maximum dose exceeded, close trial. |

3) Patients are first given a tracer amount of 10 mCi (millicuries) of $^{131}$I on a constant dose of 10 mg of ch81C6 Mab. This is termed the 'dosimetric dose' of the patient's therapy. Because of slight variations in yield and transfer of radiolabeled protein to injection syringes, the administered dose is 10 mg $\pm$ 2 mg. The administered radiation dose for the same reasons may vary 10% from the targeted dose. Patients have blood drawn for pharmacokinetic measurements after the dosimetric dose at time 6 hours, 24 hours, and every 24 hours through 7 days after the dose is given. Patients have Gamera camera imaging with whole body planar and SPECT analyses at 24 hours, and every 24 hours for 7 days. From this data, the activity of the $^{131}$I labeled ch81C6 is determined the total body exposure dose that will be delivered with the dose of millicures the patient will receive (depending on the cohort assigned) is calculated. Calculating our expected total body exposure dose from our dosimetric dose phase further assures for each patient that for this calculation we do not exceed the know tolerances of the major visceral organs as identified above.

The patients then return (approximately 1-2 weeks after the initial dose) for the potentially 'therapeutic dose' infusion. The therapeutic dose of radiolabel is on a constant dose of 10 mg of ch81 C6 Mab.

Those who experience prolonged cytopenias (as defined below) have their back-up stem cells thawed and re-infused to assist in recovery.

**Dose modifications:**

Few side effects are anticipated from infusion of the antibody, although it is possible that fever, myalgia, nausea, vomiting, urticaria, arthralgias, rigors, and shortness of breath may occur. These can be treated by repeating the diphenhydramine and acetaminophen medication regimen, as well as using 25-50 mg of meperidine intravenously. If these symptoms persist during antibody infusion, modification of infusion should occur as follows:

**Table 3: Infusion rate modifications to be followed for side effects that may occur during the administration of the antibody therapy.**

| Fever | Rigors | Shortness of Breath | % drop in diastolic blood pressure | Infusion rate adjustment |
|---|---|---|---|---|
| <39°C | Moderate | Moderate | 30-49 % | Cut rate of infusion in half |
| ≥39°C | Severe | Severe | ≥50% | Stop infusion until resolved, then restart at 50% the rate |

**Table 4: Treatment timetable for the dosimetric and therapeutic doses of I$^{113}$ labeled 81C6 monoclonal anti-tenascin antibody.**

| Time relative to dosimetric dose | Procedure |
|---|---|
| Day -2 | Start oral iodine product for thyroid protection. Base line CTs, marrows, gallium scans already done. |
| Day 0 | Dosimetric dose with 10 mCi $^{131}$I ch81C6 |
| 6 hours, 24 hours and every 24 hours until day +7 after dosimetric dose | 5 cc serum drawn for pharmacokinetics |
| 24 hours after and daily for 7 days after dosimetric dose | Gamera camera imaging for dosimetry |
| Day 7-14 after dosimetric dose | Therapeutic dose (40, 60, 80, or 100 mCi) |
| The day of the dosimetric dose | Physical exam, blood counts and chemistries |
| Daily times 3 and weekly times 4 starting after the therapeutic dose | Physical exam, blood counts and chemistries, urine analysis for proteinuria |
| Recovery from therapy (about 3 weeks after therapeutic dose) | Restaging studies |

**Measurement Of Effect**

**Toxicity:** NCI common toxicity criteria, version 2.0, are followed with a physical exam by the treatment team, complete blood count, Chem 16, LDH, Mg, and Ca for 3 days, and weekly times 4 weeks after infusion.

**Pharmacokinetics:** Blood samples are obtained at approximately 6 hours, 24 hours and daily times 7 after the trace dose of $^{131}$I - labeled ch81C6 administration to measure activity concentration in the blood as a function of time. A trichloroacetic acid (TCA) precipitation will is used to assess the protein-associated fraction of $^{131}$I in these blood samples as we have previously reported for the murine antibody 81 C6 in prior studies (Akabani G. et al., Dosimetry of 131 I labeled 81C6 monoclonal antibody administered into surgically created resection cavities in patients with malignant brain tumors. J Nucl Med. 40: 631-638, (1999)). The activity concentration of $^{131}$I in blood samples is measured in a calibrated well scintillation counter (Akabani G. et al., Dosimetry of 131I labeled 81C6 monoclonal antibody administered into surgically created resection cavities in patients with malignant brain tumors. J Nucl Med. 40: 631-638, (1999)) as previously reported. 2 milliliter aliquots are counted for $^{131}$I activity using an automated gamma counter and the percentage injected dose in the blood is calculated by comparison with standards prepared from the same administered dose.

**Dosimetry:** Planar whole body images and SPECT of the chest, abdomen, and pelvis are acquired 24 hours after infusion of the tracer dose of the radiolabeled antibody and daily for 7 days. All images are acquired on a dual head gamma camera system equipped with high energy collimators. An energy window of 15% centered on the photopeak is used in all planar and tomographic image acquisitions. SPECT acquisitions are performed in 360 degree orbits, and images are acquired in 3 degree intervals at 20 seconds per view. These projection data were acquired in 128 x 128 matrices. Standard quantitative SPECT methods are used to determine activity distributions of $^{131}$I in the liver, spleen, thyroid, and marrow (Schold SC et al., Distribution and dosimetry of I123 labeled monoclonal antibody 81C6 in patients with anaplastic glioma. Invest Radiol 28: 488-496, 1993)). The analysis of whole body images is performed using the gamma camera system computer software to generate regions of interest. This analysis yields relative clearance curves for the whole body and visceral organs (liver, spleen, marrow, thyroid gland).

The normal organ dosimetry is calculated based on standard quantitative SPECT methods for [131]I. Bone marrow dosimetry is based on the whole blood activity over time.

**Efficacy:** Patients are evaluated for efficacy upon recovery from the side effects of the therapy (anticipated at 2-4 weeks after infusion).

*Response:* Patients are re-staged (physical exam, CT scans, gallium scan, bone marrow) after therapy is completed and recovery from the side effects (anticipated to be about 3 weeks after therapy) with blood chemistries to include LDH, calcium, creatinine, TSH, $T_3$, $T_4$, total bilirubin, AST, ALT, Alkaline phosphatase, bone marrow biopsy and aspirate. Patients should have a minimum of a physical exam, LDH and CT scans performed every 6 months for 3 years for follow-up restaging.

*Criteria for response* (monitored in this trial only as a secondary goal):

Complete Response (CR): Date of initial documentation of the disappearance of all symptoms and signs of all measurable disease confirmed by physical examination, laboratory, nuclear and radiographic studies, and other non-invasive procedures repeated for all initial abnormalities or sites positive at the time of entry onto study. Accessible peripheral lymph nodes that remain suspicious must be re-biopsied to meet criterion for CR. Remissions must last for more than 4 weeks. Confirmation of remission for 4 weeks may be based on physical exam, symptoms and laboratory analyses.

Partial Response (PR): Patients who do not meet criterion for CR, however when compared with pretreatment measurements a reduction of >25% in the diameters of all measurable lesions has occurred, during which time no new lesions may appear, and no existing lesion may enlarge.

Stable Disease (SD): Patients who have a < 25% increase or decrease in the diameter of the measurable lesions, and no appearance of new lesions.

Progressive Disease (PD): Progressive disease is defined as a > or = 25% increase in the diameter of any lesion or the appearance of any new areas of malignant disease.

**On/Off-Study Criteria**

Patients remain on study until progression or withdrawal of consent and are followed for survival during their lifetime.

**Study Monitoring**

ADVERSE DRUG REACTION REPORTING:

Use the NCI Common Toxicity criteria version 2.0.

**Statistical Considerations:**

Estimate the maximum tolerated dose in terms of toxicities of $^{131}$I -labeled 81 C6 anti-tenascin human / chimeric monoclonal antibody (ch81C6) delivered intravenously in patients with lymphoma.

**Dose limiting toxicity (DLT):**

DLT is defined as a grade 3, 4, or severe or life-threatening non-hematopoietic toxicity using NCI version 2.0 criterion, or prolonged neutropenia or thrombocytopenia (as defined below*).

**[0070]** * Prolonged cytopenia: Hematopoietic toxicity consisting of more than 10 days of an ANC < 500 cells/$\mu$l despite use of G-CSF and/or the need for platelet or red cell transfusions for more than 14 days to maintain a count of >10,000 cells/$\mu$l and/or the need for red blood cell transfusions to maintain a hematocrit >25% will be considered 'prolonged' and a dose limiting toxicity, unless it is due to the underlying disease as documented by the treating physician.

**Maximum Tolerated Dose (MTD):** The maximum tolerated dose in this trial is the dose of radiolabeled antibody that producers $\leq$ 2 of 6 patients experiencing a dose limiting toxicity.

**Radiation dose escalation plan:**

Four dose levels will be considered: 40 mCi, 60 mCi, 80 mCi, 100 mCi. Standard phase I dose escalation as outlined below will be used to estimate the maximum tolerated dose.

Patients are enrolled and treated in a standard phase I manner with 2 patients minimum (prior to escalating the dosage) planned for each cohort of radiolabel dosing. If zero patients experience dose limiting toxicity at a certain dose, the dose will be escalated to the next level. If 1 patient experiences dose limiting toxicity of the first 3, then 3 more will be entered at the same level. If $\leq$ 2 of these 6 patients experience a dose limiting toxicity, then we will escalate to the next cohort. However, if 2 or 3 patients of the first 3 patients experience dose limiting toxicity, then the maximum tolerated dose has been exceeded and this trial will be closed. The maximum tolerated dose will be estimated as the next lower dose of radiolabel in this trial (the highest dose given that did not exceed the maximum tolerated dose).

In order to decrease undesirable side effects related to non-specific uptake of the radiolabeled antibody in normal tissues, an expanded protocol is used. This protocol allows the estimation of the optimal dose of unlabeled anti-tenascin antibody to deliver prior to infusion of the labeled dose. After determination of the maximum tolerated dose, the standard phase I trial is expanded to include a rapid infusion over 5 -10 minutes of unlabeled antibody immediately prior to the infusion of a labeled dose of antibody. The expanded trial is a dose escalation study in standard fashion of the unlabeled antibody dose in 4 cohorts of differing antibody doses: 100 mg, 200 mg, 400 mg and 800 mg.

**Table 5**

| Radiolabeled antibody dose | Number treated | Number experiencing dose limiting toxicity | Action |
|---|---|---|---|
| 40 mCi | 3 | 0 | Escalate to next cohort |
| | | 1 | Continue accrual |
| | | 2 | Maximum dose exceeded. Close trial and get approval to deescalate dose |
| 40 mCi | 6 | 1 or 2 | Advance to next cohort |
| | | 3 or more | Maximum dose exceeded. Close trial and get approval to deescalate dose |
| 60 mCi | 3 | 0 | Escalate to next cohort |
| | | 1 | Continue accrual |
| | | 2 | Maximum dose exceeded, close trial |
| 60 mCi | 6 | 1 or 2 | Advance to next cohort |
| | | 3 or more | Maximum dose exceeded, close trial |
| 80 mCi | 3 | 0 | Escalate to next cohort |
| | | 1 | Continue accrual |

(continued)

| Radiolabeled antibody dose | Number treated | Number experiencing dose limiting toxicity | Action |
|---|---|---|---|
| | | 2 | Maximum dose exceeded, close trial |
| 80 mCi | 6 | 1 or 2 | Advance to next cohort |
| | | 3 or more | Maximum dose exceeded, close trial |
| 100 mCi | 3 | 0 | Escalate to next cohort |
| | | 1 | Continue accrual |
| | | 2 | Maximum dose exceeded, close trial |
| 100 mCi | 6 | 1 or 2 | Maximum rec. dose |
| | | 3 or more | Maximum dose exceeded, close trial. |

**Evaluate the pharmacokinetics and dosimetry of radiolabeled 81C6 anti-tenascin human/chimeric monoclonal antibody with differing doses radiolabel.**

Pharmacokinetics are monitored as above. The results are used to model the elimination of the antibody from the blood based on a compartmental model previously developed (Birchall A. and James AC. *A microcomputer algorithm for solving first-order compartmental involving recycling.* Health Physics **56**:857-868, 1989)).

Dosimetry to the whole body, tumor, and visceral organs is assessed as above. The results are used in a non-cycling compartmental model for all the organs. Serial whole body and SPECT images indicate the transfer between organs and tumor sites. This approach has previously been described (Birchall A. and James AC. A microcomputer algorithm for solving first-order compartmental involving recycling. Health Physics 56:857-868, 1989)).

These data on dosimetry and pharmacokinetics will be collected in conjunction with toxicity data on all patients. This information allows the determination of the whole body exposure doses for each cohort of patients in this trial.

**Evaluate the potential anti-lymphoma effects of radiolabeled ch81C6.**

Patients are evaluated for response by physical exam, blood work, CT scans and bone marrow exam upon recovery from the therapy. Best response is documented as above.

Kaplan - Meier estimates of progression free and overall survival are used, however, this is primarily a phase I trial and comparisons with historical controls are not planned.

**EXAMPLE 3**

**Clinical Trial Results**

The following table summarizes the results of a phase I clinical trial involving two patients. The clinical trial was carried out according to protocol described in Example 2, however, no cold blocking antibody was given prior to labeled dose in this phase I trial. Patients with relapsed or refractory NHL who are not candidates for high dose therapy, have not been previously radiated to tissue tolerance, do not have >25% marrow involvement with disease, and have normal blood counts and adequate liver/renal function were eligible for this trial. The first patient had refractory well-differentiated lymphoma following three different chemotherapy and rituximab regimens without any significant response. The second patient had diffuse large cell lymphoma refractory to 3 standard regimens of chemotherapy. Table 6 below shows the whole-body, visceral organ and tumor dosimetry.

**Table 6**

| | Patient #1 | | Patient #2 | |
|---|---|---|---|---|
| | Long term effective | Absorbed | Long term effective | Absorbed |
| | Half life (hr) | Dose (cGy) | Half life (hr) | Dose (cGy) |
| Whole Body | 116 | 58 | 109 | 70 |
| Blood | 90 | 34 | 168 | 198 |
| Liver | 94 | 297 | 69.9 | 459 |
| Marrow | 64 | 263 | 64 | 404 |
| Tumor | 192 | 1594 | 168 | 1258 |

The table reveals that even without a cold dose for blocking of non-specific uptake, the tumor still concentrates the radiolabeled antibody at a ratio of 5X over visceral organs. Each patient noted 1 night sweat and mild diarrhea the night of therapy and low-grade fever persisting for a few days. Both patients experienced transient myelosuppression occurring between weeks 4-6 from therapy. With early follow up of 1-3 months, both patients have responded with decreased tumor size.

These results, as well as the clinical outcomes for the patients, illustrate that the targeted radiotherapy of the present invention is useful in treating relapsed lymphoma patients or those incompletely responding to conventional chemotherapy regimens or rituximab therapy. Additionally, the targeted radiotherapy of the present invention has applicability in treating other tumors such as breast cancer, lung cancer, gastrointestinal cancers (e.g., colon cancer, stomach cancer) and other tumors that may express tenascin.

The foregoing is illustrative of the present invention, and is not to be construed as limiting thereof. The invention is defined by the following claims.

**Claims**

1. Use of an antibody that binds to tenascin, for the manufacture of a medicament for the treatment of Non-Hodgkins lymphoma by parenteral administration;
   the antibody being selected from monoclonal antibody 81C6 and antibodies that bind to the epitope bound by monoclonal antibody 81C6; and
   the antibody being coupled to a radioisotope.

2. Use according to claim 1, wherein said Non-Hodgkin's lymphoma is unresponsive to chemotherapy treatment selected from the group consisting of rituximab, cyclophosphamide, doxorubicin, vincristine and prednisone treatment.

3. Use according to claim 1, wherein said Non-Hodgkin's lymphoma is unresponsive to rituximab treatment.

4. Use according to claim 1, wherein said radioisotope is selected from the group consisting of $^{131}$I, $^{90}$Y, $^{211}$At, $^{212}$Bi, $^{67}$Cu, $^{186}$Re, $^{188}$Re, and $^{212}$Pb.

5. Use according to claim 1, wherein said radioisotope is $^{131}$I.

6. Use according to claim 1, wherein said antibody coupled to a radioisotope is administered in an amount of from 50 to 1000 Gy (5,000 rads to 100,000 rads).

**Patentansprüche**

1. Verwendung eines Antikörpers, der sich an Tenascin bindet, für die Herstellung eines Medikaments für die Behandlung von Non-Hodgkin-Lymphom durch parenterale Verabreichung;
   wobei der Antikörper unter monoklonalem Antikörper 81C6 und Antikörpern ausgewählt wird, die sich an das Epitop binden, der durch den monoklonalen Antikörper 81C6 gebunden wird; und
   der Antikörper an ein Radioisotop gekoppelt ist.

**2.** Verwendung nach Anspruch 1, wobei das Non-Hodgkin-Lymphom auf eine Chemotherapiebehandlung ausgewählt aus der Gruppe bestehend aus Rituximab-, Cyclophosphamid, Doxorubicin-, Vincristin- und Prednison-Behandlung nicht anspricht.

**3.** Verwendung nach Anspruch 1, wobei das Non-Hodgkin-Lymphom auf die Rutuximab-Behandlung nicht anspricht.

**4.** Verwendung nach Anspruch 1, wobei das Radioisotop aus der Gruppe ausgewählt ist bestehend aus $^{131}$I, $^{90}$Y, $^{211}$At, $^{212}$Bi, $^{67}$Cu, $^{186}$Re, $^{188}$Re und $^{212}$Pb.

**5.** Verwendung nach Anspruch 1, wobei das Radioisotop $^{131}$I ist.

**6.** Verwendung nach Anspruch 1, wobei der Antikörper, der an ein Radioisotop gekoppelt ist, in einer Menge von 50 bis 1000 Gy (5.000 Rad bis 100.000 Rad) verabreicht wird.

**Revendications**

**1.** Utilisation d'un anticorps qui se lie à la ténascine, pour la fabrication d'un médicament pour le traitement de lymphomes non hodgkiniens par une administration parentérale;
l'anticorps étant choisi parmi l'anticorps monoclonal 81C6 et des anticorps qui se lient à l'épitope lié par l'anticorps monoclonal 81C6; et
l'anticorps étant couplé à un radio-isotope.

**2.** Utilisation suivant la revendication 1, dans laquelle lesdits lymphomes non hodgkiniens sont insensibles à un traitement de chimiothérapie choisi dans le groupe constitué d'un traitement par rituximab, cyclophosphamide, doxorubicine, vincristine et prednisone.

**3.** Utilisation suivant la revendication 1, dans laquelle lesdits lymphomes non hodgkiniens sont insensibles à un traitement par rituximab.

**4.** Utilisation suivant la revendication 1, dans laquelle ledit radio-isotope est choisi dans le groupe constitué de $^{131}$I, $^{90}$Y, $^{211}$At, $^{212}$Bi, $^{67}$Cu, $^{186}$Re, $^{188}$Re et $^{212}$Pb.

**5.** Utilisation suivant la revendication 1, dans laquelle ledit radio-isotope est $^{131}$I.

**6.** Utilisation suivant la revendication 1, dans laquelle ledit anticorps couplé à un radio-isotope est administré dans une quantité de 50 à 1000 Gy (de 5.000 rads à 100.000 rads).

## REFERENCES CITED IN THE DESCRIPTION

### Patent documents cited in the description

- US 60257108 B
- US 5624659 A, Bigncr and Zalutsky
- US 6624659 B
- US 4474893 A
- US 4816567 A, Cabilly
- US 4676980 A, Segel

### Non-patent literature cited in the description

- **RIZZIERI et al.** *Blood,* 15 November 1999, vol. 94 (10
- Classification Project. National Cancer Institute sponsored study of classification of Non-hodgkin's lymphomas. *Cancer,* 1982, vol. 49, 2112-2135
- **M. WALKER et al.** *Molec. Immunol.,* 1982, vol. 26, 403-11
- **W. HUSE.** *Science,* 1989, vol. 246, 1275-81
- **M. BOURDON et al.** *Cancer Res,* 1983, vol. 43, 2796
- Monoclonal Antibodies and Cancer Therapy. Alan R. Liss Inc, 1985
- **M. ZALUTSKY ; A. NARULA.** *Appl. Radiat. Isot.,* 1987, vol. 38, 1051
- Remington, The Science And Practice of Pharmacy. 1995
- **AKABANI G. et al.** Dosimetry of I labeled 81C6 monoclonal antibody administered into surgically created resection cavities in patients with malignant brain tumors. *J Nucl Med,* 1999, vol. 40, 631-638
- **AKABANI G. et al.** Dosimetry of I labeled 81C6 monoclonal antibody administered into surgically created resection cavities in patients with malignant brain tumors. *J Nucl Med.,* 1999, vol. 40, 631-638
- **SCHOLD SC et al.** Distribution and dosimetry of I123 labeled monoclonal antibody 81C6 in patients with anaplastic glioma. *Invest Radiol,* 1993, vol. 28, 488-496
- **BIRCHALL A. ; JAMES AC.** A microcomputer algorithm for solving first-order compartmental involving recycling. *Health Physics,* 1989, vol. 56, 857-868